# EUROPEAN PATENT SPECIFICATION

(11) **EP 0 387 265 B1**
(45) Date of publication and mention of the grant of the patent: **21.04.1993**
(21) Application number: 88909305.0
(22) Date of filing: 13.10.1988
(51) Int. Cl.: A61K 31/60, C07C 229/60

(54) **USE OF 5-AMINOSALICYCLIC ACID (5-ASA) FOR THE PRODUCTION OF A PHARMACEUTICAL PREPARATION FOR USE IN REPERFUSION TREATMENT**
VERWENDUNG VON 5-AMINOSALICYLSÄURE (5-ASA) ZUR HERSTELLUNG EINER ARZNEIMITTELZUBEREITUNG ZUR VERWENDUNG BEI EINER REPERFUSIONSBEHANDLUNG
UTILISATION D'ACIDE 5-AMINOSALICYLIQUE (5-ASA) POUR LA PRODUCTION D'UNE PREPARATION PHARMACEUTIQUE DESTINEE A ETRE UTILISEE DANS UN TRAITEMENT PAR REPERFUSION

(30) Priority: 14.10.1987 DK 5369/87
(43) Date of publication of application: 19.09.1990
(73) Proprietor: FARMACEUTISK LABORATORIUM FERRING A/S, DK-2720 Vanlose (DK)
(72) Inventor: BONDESEN, Stig, DK-5000 Odense C (DK); HALSKOV, Soren, DK-2830 Virum (DK)
(74) Representative: Christensen, Bent
(86) International application number: DK8800166
(87) International publication number: WO8903216

(56) References cited:
- US-A- 4 496 553
- Chemical Abstracts, vol. 103 (1985), abstract no. 134760b, Circ. Res. 1985, 56(6), 895-898 (Eng.)
- Chemical Abstracts, vol. 103 (1985), abstract no. 158430v, J. Free Radicals Biol. Med. 1985, 1(2), 103-110 (Eng.)
- Chemical Abstracts, vol. 105 (1986), abstract no. 35140j, J. Free Radicals Biol. Med. 1985, 1(4), 273-280 (Eng.)
- Chemical Abstracts, vol. 107(1987) abstract no. 126711d, Biochem. Pharmacol. 1987, 36(15), 2467-2472 (Eng.)
- GUT, 1987, 28: 196-200, Myers B. et al.
- DIGESTIVE DISEASE ANDS SCIENCES (1991), S. Bondensen et al.
- CLIN. PHARMACOKINETICS, 10: 285-302 (1985), U. Klotz
- INFLAMMATORY BOWEL DISEASE, ed. G. Järnerot, Raven Press, N.Y.(1987), 153-164, G. Järnerot
- BRIT. MED. JOURN. 269 (1988): 1442, Novis et al.
- DRUGS 37, 73-86 (1989), G. Järnerot

## Description

The invention concerns the use of 5-aminosalicylic acid (5-ASA) for production of a pharmaceutical preparation for use in reperfusion treatment in connection with coronary circulation diseases and their complications, such as coronary sclerosis, coronary thrombosis, variant angina, unstable angina and myocardial infarct of any origin, partly as acute intervening treatment and partly as prophylactic treatment, including prophylaxis in thrombolytic treatment, angioplastic and coronary by-pass operation. 5-aminosalicylic acid has till now preferably been used as an active component in certain drugs for treatment of colitis ulcerosa and Crohn's disease, but the compound has now surprisingly been found also to be active against mechanisms which are important for tissue injuries in the heart musculature (reperfusion ischemia).

Free oxygen radicals are highly toxic substances, which appear to be abundantly formed in ischemic tissue, and which are therefore increasingly considered to be pathogenic factors in many forms of tissue injuries (McCord, New England J. Med. 312 (3), 159 (1985)). Particularly great attention is paid to the superoxide radical 0^{-.}₂, which is formed by reducing the dioxygen molecule with one electron. The superoxide radical is a good reducing agent and a reasonably good oxidizing agent, and it is capable of initiating chain reactions. Free oxygen radicals, including the superoxide radical, are e.g. involved in injuries of the heart musculature and of the intestinal mucosa (Craven et al., Gastroenterology 92, 1978 (1987)), but it has been difficult to elucidate the exact nature of such injuries. The function of the free oxygen radicals has been examined in several cardiological experimental models, and it has been found that an ischemic heart can be protected against reperfusion injuries if the free radicals of the oxygen which occur in ischemic tissue are removed; see Werns et al., Circulation Research 56 (6), 895 (1985) and Otani et al., J. Surgical Research 41, 126-133 (1986). It also seems to be of decisive importance for survival of threatened heart tissue in connection with coronary thrombosis that free oxygen radicals are formed, and that these are eliminated with a view to limitation of the size of the damage; see Lancet, 29th October 1983, page 1010. Several examinations of the so-called reperfusion ischemia in animal hearts have shown that removal of the superoxide radicals by means of the enzyme superoxide dismutase (SOD), which specifically acts by catalyzing the dismutation of the super-oxide radical to hydrogen peroxide and oxygen according to the scheme

O^{-.}₂ + O^{-.}₂ + 2H⁺ → H₂O₂ + O₂

causes significant reduction in the extent of the damage to the heart tissue; see Ambrosio et al., Circulation 74 (6), 1424 (1986). However, when treating diseases in humans, this mode of treatment involves administration of more or less foreign protein in the blood path, which in turns involves a risk of immunization and allergy. Further, no effect outside the blood path can be expected.

The enzyme xanthine oxidase occurs in high concentrations in ischemic tissue and is possibly responsible for the production of most of the amount of oxygen radicals which occur in the tissue in connection with the conversion of hypoxanthine to xanthine; see J. Mol. Cell. Cardiol. 15, 713 (1983). The enzyme xanthine oxidase may be inhibited by means of the compound allopurinol, but this principle of treatment is only effective against free radicals which are formed by the reactions of the oxidase, and theoretically it will be a slow effect. Such a treatment cannot either counteract free radicals already formed and cannot stop chain reactions in progress.

Increasing interest is attached to elucidating the mechanisms in connection with reperfusion ischemic injuries, in particular because these years there is an increasing acceptance of acute treatment of coronary thrombosis with thrombolysis (streptokinase, thromboblastin) and mechanical expansion of coronary occlusions (angioplastic); see Collen, Circulation 72, 18 (1965) and Pepine el al., Am. Heart J. 197, 820 (1984). It is uncertain to what extent reperfusion injuries occur, but in view of the results obtained till now in animal experiments the occurence of reperfusion injuries must be considered a realistic risk that can counteract the otherwise good results of the new mode of treatment.

It is an open question whether similar mechanisms are involved in connection with myocardial infarct in "spontaneous healing" under the conditions where it is attempted to improve the regional bleading with the mechanisms of the organism.

Apparently, free radicals of oxygen can be formed under all conditions, also at very low oxygen concentrations (Hall, E. R., The Oxygen Effect, in Hall E. T. (red.) Radiobiology for the Radiologist, 48-61, Harper & Row, New York, (1973)), and these can contribute to the extent of the damage that result.

Recognizing that reperfusion of ischemic myocardium is connected with the formation of free oxygen radicals and that these radicals influence the final extent of the irreversible reperfusion injury, the effect of N-2-mercaptopropionyl glycine (MPG) has been examined with a view to limiting the infarct size after ischemia/reperfusion in dogs (Mitsos et al., Circulation 73 (5), 1077 (1986)). Administration of MPG together with neutrophilic antiserum reduced the infarct size by 63% compared to a control group which received non-immune serum. However, the results relate to an experimental animal model of acute myocardial ischemia and reperfusion injuries, and it is realized that no conclusions may be drawn with respect to the useful effects in intervention of longer duration.

The compound 5-aminosalicylic acid is an independently effective agent in inflammatory intestinal diseases which has been used for more than 40 years in the form of the compound sulfasalazine (5-(p-(2-pyridylsulfamoyl)phenylazo) -salisylic acid). The latter compound is cleaved in the organism under formation of 5-ASA and sulfapyridine according to the scheme 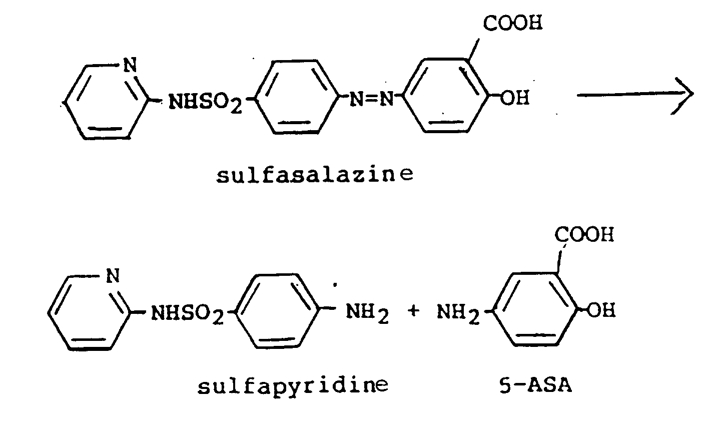
Recent years have seen the development of modes of treatments with peroral or rectal administration of the pure substance, i.e. non-bound 5-ASA. No side effects have been observed which can be related to the substance with certainty. Intravenous administration is not immediately topical in inflammatory intestinal diseases, and skilled persons have actually had a prejudice against using 5-ASA in connection with intravenous treatment. Thus, to date no results have been published which can elucidate the pharmacokinetics of the compound in intravenous administration, and further no intraveous forms of 5-ASA are known (V. Klotz, Clin. Pharmacokin. 10, 285-302 (1985)). One of the reasons why it has not been interesting to administer 5-ASA into the blood path is that the compound has long been suspected of nephrotoxicity because nephrotoxic effect has been demonstrated in tests with rats (I. C. Calder et al., Brit. J. Med. 1, 152-154 (1972)). New tests performed on humans indicate, however, that 5-ASA in moderate doses does not have any noticeable nephrotoxic effect on humans (S. Bondesen et al., Acta. Med. Scand. 221, 227-242 (1987)). Metabolization of 5-ASA has also been an argument against intravenous use of the compound, but recently 5-ASA has been used in two pharmacokinetic examinations where no side effects were found at single doses of 100-500 mg i.v. Incidentally, the substance was found to be metabolized and separated rapidly. Either 100 or 250 mg of 5-ASA were administered intravenously to 6 normal test humans, and no side effects or changes were observed in the liver or kidney function. The substance was eliminated rapidly (half-life 35 minutes), predominantly by metabolization to acetyl-N-5-ASA and to some degree by separation in the kidneys by filtration or secretion. The substance was rapidly distributed after administration in a distribution volume of 0.4 l/kg, which means that the substance gets outside the blood path in the tissue fluids (S. Bondesen et al., 1987, being printed). The concentration of 5-ASA in the blood path was at a maximum about 10 to 15 times higher than the concentrations which are observed in treatment of inflammatory intestinal diseases with 5-ASA in tablet form. Further, 500 mg of 5-ASA have been administered to 6 test humans without observable side effects; see B. Myers et al., Gut 28, 196-200 (1987).

The effect of 5-ASA in connection with prevention of reperfusion injuries is to be related to the above-mentioned superoxide radicals which are formed by phagocytes in the organism, and which can initiate chain reactions if they are not kept under control; see McCord, Science 185, 529 (1975), and Ahnfelt-Rønne and Haagen Nielsen, Agents and Actions 516 (1986). In the organism, there is therefore a need for a compound capable of catching and destroying such superoxide radicals and other reactive oxygen radicals, and in fact a plurality of protective systems is active in the cells with the purpose of preventing formation of free radicals or removal of excess amounts of formed free radicals. These systems are known as preventive and chain-destructive antioxidants, see Burton and Ingold, Science 224, 569 (1984). The first group comprises i.a. catalase, glutathione peroxidase and superoxide dismutase (SOD). The second group consists of the chain-destructive antioxidants, the so-called radical scavengers which are capable of transferring hydrogen to free radicals and simultaneously stabilizing the resulting new free radical. This group comprises the physiological antioxidants ( α-tocopherol, ascorbic acid and β-carotene), phenols and certain arylamines.

In contrast to the structural analogous 4-aminosalicylic acid (4-ASA), 5-aminosalicylic acid has been found to belong to the group of radical scavengers. The effect of 5-ASA has still not been explained, but has i.a. been related to the arachidonic acid metabolism (Peppercorn, Intern. Med. 3, 377 (1984)). Its possible importance in the inflammatory process in the intestine, against which 5-ASA has been used for many years, as mentioned, has not been elucidated, however. It has been shown clearly in recent years that 5-ASA has a pronounced effect as a radical scavenger, partly by physical methods (Ahnfelt-Rønne and Haagen Nielsen, supra) and chemical methods (Grisham et al., Gastroenterology 92 (5), 1416 (1987)), and partly by biological in vitro tests (Miyachi et al., Gut 28, 190 (1987) and Craven et al., supra). Hereby, it has been found in an unambiguous and convincing manner that 5-ASA - in contrast to sulfasalazine, sulfapyridine and the closely related 5-ASA analogous acetylsalicylic acid and sodium salicylate, both of which have no effect against colitis ulcerosa - is a radical scavenger capable of neutralizing the toxic properties of the free radicals of oxygen and stopping chain reactions in progress. The effect of 5-ASA is quite instantaneous.

It has been found that 5-ASA can protect the gastric mucosa against reperfusion ischemic injury (Smith et al., Gastroenterology 92 (5), 1647 (1987)), which was shown when the two cleavage products of sulfasalazine (5-ASA and sulfapyridine) were examined in a test with rats. It was found that 5-ASA, but not sulfapyridine, offers protection against reperfusion ischemic injury in the gastric mucosa, and it is assumed on the basis of this observation that 5-ASA will be of significant therapeutic value in the treatment of gastric ulcers in humans.

In view of the foregoing, it has now been found that 5-aminosalicylic acid in suitable formulations can be used in reperfusion treatment of coronary thrombosis and coronary sclerosis (coronary by-pass operation), just as the compound can be used in acute intervention as well as in prophylactic treatment against myocardial infarct. The usefulness of the substance for these purposes is primarily attributed to its well-documented effect as radical scavenger, but may also be attributed to the absence of important side effect profile and its ability to be administered intravenously. Intravenous administration of 250 mg of 5-ASA to humans having normal coagulation has not been found to result in any effect of the thrombocyte function or fibrinolysis in humans (in contrast to what is observed by administration of acetylsalicylic acid); cf. K. Winther, S. Bondesen and E. Hvidberg, 1987, being printed in Eur. J. Clin. Pharmacol.). This fact also opens up the possibility of using 5-ASA without interference with anti-aggregatory or thrombolytic treatment.

The 5-ASA modes of administration are to aim at plasma levels of 5-ASA of between 5 and 20_{/}ug/ml, possibly higher if toxicological examinations prove that this involves no risk, and also if it should be necessary in special situations.

For acute intervention treatment, the substance is to be administered intravenously, either intermittently or as infusion, possibly for some days.

For this use, the substance is to be dispersed as a dry matter in vials or ampoules with associated solvent liquid in the form of a neutral buffer with optional necessary preserving agents. The measured amount can optionally depend upon whether the administration mode is intermittent or continuous as infusion.

For "subacute" intervention, e.g. rapidly after the intravenous treatment, it is considered expedient to administer the substance orally. Here, intermittent treatment as well as continuous treatment may likewise be used. For intermittent peroral treatment, a tablet with immediate and total release of the substance in the upper small intestine may be used. 5-ASA will be absorbed from this preparation and be distributed almost just as rapidly as in intravenous administration, depending of course upon the depletion rate of the stomach.

For more continuous peroral treatment with the purpose of more constant and controlled values of 5-ASA in the blood path, e.g. with a view to prophylactic treatment, a slow release tablet may be used, which releases 5-ASA at a fixed constant rate during the passage through the small intestine, so that all the substance will be released when the tablet reaches the large intestine. Here the absorption of 5-ASA is poor.

Infusion via a probe in the small intestine, either of an intravenous preparation or another 5-ASA solution, may moreover be practically useful in certain situations.

Rectal administration modes may optionally also be considered.

## Claims

1. Use of the compound 5-aminosalicylic acid (5-ASA) for the production of a pharmaceutical preparation for use in reperfusion treatment in connection with coronary circulation diseases and their complications, such as coronary sclerosis, coronary thrombosis, variant angina, unstable angina and myocardial infarct of any type, partly as acute intervening treatment and partly as prophylactic treatment, including prophylaxis in thrombolytic treatment, angioplastic and coronary by-pass operation.

2. Use according to claim 1, **characterized** by producing a preparation for use in intervention, said preparation being dispensed as a dry matter in vials or ampoules with associated solvent liquid in the form of a neutral buffer with optional preserving agents.

3. Use according to claim 1, **characterized** by producing a preparation for oral administration, preferably in the form of a tablet with immediate and total release of 5-ASA in the upper small intestine.

4. Use according to claim 1, **characterized** by producing a preparation in the form of a tablet which slowly releases 5-ASA during the passage through the small intestine.

5. Use according to claim 1, **characterized** by producing an infusion solution containing 5-ASA for administration via a probe in the small intestine.

## Patentansprüche

1. Verwendung der Verbindung 5-Aminosalicylsäure (5-ASA) zur Herstellung einer pharmazeutischen Zubereitung zur Verwendung bei einer Reperfusionsbehandlung in Verbindung mit Koronardurchblutungskrankheiten und deren Komplikationen, wie z.B. Koronarsklerose, Koronarthrombose, variante Angina, instabile Angina und myokardiale Infarkte jeden Typs, teilweise als akute Interventionsbehandlung und teilweise als prophylaktische Behandlung, einschließlich der Prophylaxe bei thrombolytischen Behandlungen, Angioplastik- und koronaren Bypass-Operationen.

2. Verwendung nach Anspruch 1, gekennzeichnet durch das Herstellen einer Zubereitung zur Verwendung bei der Intervention, wobei die Zubereitung als trockener Stoff in Fläschchen oder Ampullen zusammen mit einer Lösemittelflüssigkeit in Form eines neutralen Puffers mit optionalen Konservierungsstoffen dispensiert wird.

3. Verwendung nach Anspruch 1, gekennzeichnet durch die Herstellung einer Zubereitung für die orale Verabreichung, vorzugsweise in Form einer Tablette mit sofortiger und vollständiger Freisetzung von 5-ASA im oberen Dünndarm.

4. Verwendung nach Anspruch 1, gekennzeichnet durch die Herstellung einer Zubereitung in Form einer Tablette, welche 5-ASA langsam während des Durchgangs durch den Dünndarm freisetzt.

5. Verwendung nach Anspruch 1, gekennzeichnet durch die Herstellung einer Infusionslösung, welche 5-ASA zur Verabreichung über eine Sonde im Dünndarm enthält.

## Revendications

1. Utilisation de l'acide 5-aminosalicylique [5-ASA] pour la production d'une préparation pharmaceutique utilisable dans le traitement de reperfusion en relation avec des maladies de la circulation coronaire et de leurs complications, telles que sclérose coronaire, thrombose coronaire, angine variable, angine instable et infarctus du myocarde d'origine quelconque, en partie en tant que traitement aigu d'intervention et en partie en tant que traitement prophylactique, incluant la prophylaxie dans un traitement thrombolytique, une opération angioplastique et de pontage coronaire.

2. Utilisation suivant la revendication 1, caractérisée par la production d'une préparation utile en intervention, cette préparation étant distribuée en tant que matière sèche dans des flacons et des ampoules avec un liquide solvant associé sous la forme d'un tampon neutre avec des agents de conservation facultatifs.

3. Utilisation suivant la revendication 1, caractérisée par la production d'une préparation pour l'administration orale, de préférence sous la forme d'un comprimé avec libération immédiate et totale du 5-ASA dans le petit intestin supérieur.

4. Utilisation suivant la revendication 1, caractérisée par la production d'une préparation sous la forme d'un comprimé qui libère lentement le 5-ASA pendant le passage à travers le petit intestin.

5. Utilisation suivant la revendication 1, caractérisée par la production d'une solution de perfusion contenant le 5-ASA pour l'administration par l'intermédiaire d'une sonde dans le petit intestin.
